# EUROPEAN PATENT APPLICATION

(11) **EP 1 547 624 A1**
(43) Date of publication of application: **29.06.2005**
(21) Application number: 03794261.2
(22) Date of filing: 05.09.2003
(51) Int. Cl.: A61L 9/16, A61L 9/22, A61L 9/014

(54) **AIR PURIFIER**

(30) Priority: 05.09.2002 JP 2002259867
(71) Applicant: Sanei Kensetsu Kabushiki Kaisha, Tsuru-shi, Yamanashi 402-0025 (JP)
(72) Inventor: OSADA, Tasuku, Kofu-shi, Yamanashi 400-0862 (JP); KOMATSU, Toru Sanei Kensetsu Kabushiki Kaisha, Tsuru-shi, Yamanashi 402-0025 (JP); NISHIMAKI, Ryusuke Sanei Kensetsu Kabushiki Kaisha, Tsuru-shi, Yamanashi 402-0025 (JP)
(74) Representative: Thomas, Philip John Duval
(86) International application number: PCT/JP2003/011378
(87) International publication number: WO 2004/022113

(57) **Abstract**

An air purifier having a high level of air purifying power, especially an air purifier capable of efficiently removing airborne dust, smoke from cigarette or cigar or tobacco, harmful gas and the like, is disclosed. This air purifier comprises at least a discharge electrode (1) capable of producing ions upon discharge, a counter electrode (2) located opposite to the discharge electrode, and a power supply (3) capable of applying voltage across the discharge electrode and the counter electrode for inducing discharge for producing ions at the discharge electrode. The counter electrode (2) comprises a gas absorbent material comprising: a high-temperature carbonized charcoal which has been carbonized at a temperature of about 800°C or above; a low-temperature carbonized charcoal which has been carbonized at a temperature of about 500°C or below; and alginic acid or its salt or calcium oxide.

## Description

### TECHNICAL FIELD

The present invention relates to an air purifier, especially an ionic wind-type air purifier, which can remove airborne dust, smoke from cigarette or cigar or tobacco, harmful gas and the like.

### BACKGROUND ART

An ionic wind-type air purifier employs the ionic wind which is produced by applying a high voltage of about 5 to 10 kV between electrodes for corona discharge to ionize fine particles. More specifically, the ionized particles are moved by an electric field between the electrode to produce an ionic wind through which dust or smoke from cigarette or cigar or tobacco in the air is adsorbed on a dust collecting electrode to purify the air. Air purifiers of this type are disclosed, for example, in Japanese Patent Laid-Open Nos. 172190/1985 and 140657/1987.

While a metal such as aluminum is used as the dust collecting electrode in such air purifiers, Japanese Patent Laid-Open No. 199653/1998 discloses that a coating of activated carbon is utilized as a surface of an electrode located opposite to a corona discharge electrode. The apparatus disclosed in this laid-open publication, however, is an ionic wind generator utilizing
negative ions in which ozone involved in corona discharge is removed by taking advantage of the activated carbon.

Charcoal such as activated carbon is well known to have a material adsorptive capability. Further, Japanese Patent Laid-Open No. 226207/2000 discloses a production process of an activated wood charcoal having both a low-temperature carbonized part and a high-temperature carbonized part. This production process comprises the steps of: heat-treating wood chips at 450 to 550°C to carbonize the wood chips (a low-temperature carbonization step); and subsequently heat-treating the carbonized wood chips at 800 to 900°C to further carbonize the wood chips (a high-temperature carbonization step). In this publication, however, there is no specific disclosure on any molded product using this activated wood charcoal. Further, this publication neither suggests nor discloses any useful binder for molding.

### SUMMARY OF THE INVENTION

The present inventors have now found that the use of a carbon molding, which has been carbonized at a specific temperature, as a dust collecting electrode in an ionic wind-type air purifier can realize an air purifier having a very high level of air purifying power. More specifically, it has been found that, when a gas absorbent material produced by combining a high-temperature carbonized charcoal, which has been carbonized at a temperature of about 800°C or above, a low-temperature carbonized charcoal, which has been carbonized at a temperature of about 500°C or below, and alginic acid or calcium oxide is used as an electrode, an air purifier capable of efficiently removing airborne dust, smoke from cigarette or cigar or tobacco, harmful gas and the like can be realized.

Accordingly, an object of the present invention is to provide an air purifier having a high level of air purifying power.

The above object can be attained by an air purifier comprising at least a discharge electrode capable of producing ions upon discharge, a counter electrode located opposite to the discharge electrode, and a power supply capable of applying voltage between the discharge electrode and the counter electrode for inducing discharge for producing ions at the discharge electrode, the counter electrode comprising a gas absorbent material comprising: a high-temperature carbonized charcoal which has been carbonized at a temperature of about 800°C or above; a low-temperature carbonized charcoal which has been carbonized at a temperature of about 500°C or below; and alginic acid or its salt or calcium oxide.

### BRIEF DESCRIPTION OF THE DRAWINGS

Fig. 1 is a diagram showing the basic construction of the air purifier according to the present invention;
Fig. 2 is a diagram showing an air purifier used in the working example; and
Fig. 3 is a diagram showing the basic construction of an experimental apparatus used in the working example.

### DETAILED DESCRIPTION OF THE INVENTION

### Basic construction of air purifier

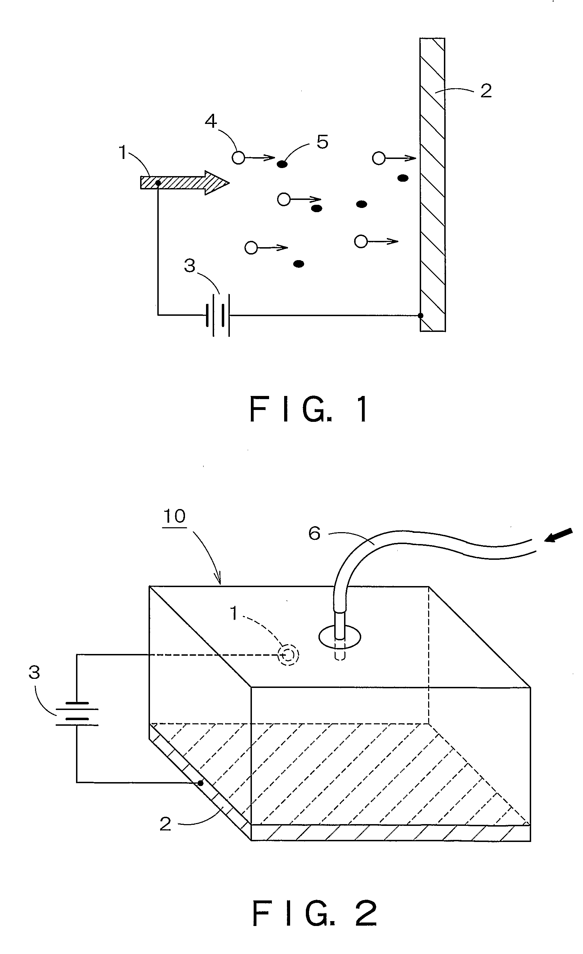

The air purifier according to the present invention basically comprises a discharge electrode capable of producing ions upon discharge, a counter electrode located opposite to the discharge electrode, and a power supply capable of applying voltage between the discharge electrode and the counter electrode for inducing discharge for producing ions at the discharge electrode. Fig. 1 is a typical diagram showing this basic construction. In this drawing, a discharge electrode 1 is in the form of a needle formed of, for example, carbon, platinum, tungsten, gold, silver, titanium, stainless steels, nickel, tantalum, copper, phosphor bronze, a metal plated wire, or a nickel plated wire. In this discharge electrode, discharge capable of producing ions is carried out. In the present invention, discharge at the discharge electrode is the so-called "gaseous discharge" which includes corona discharge, glow discharge, and arc discharge.

An electrode 2 is provided opposite to the discharge electrode 1. The counter electrode 2 comprises a gas absorbent material which will be described later. Voltage for inducing discharge in the discharge electrode 1 is applied from a power supply 3 across the two electrodes. The voltage may be properly determined. When the discharge is corona discharge, however, the voltage is generally, for example, about 4000 to 10000 V.

An electric field is converged on the front end part of the discharge electrode 1 to which a negative voltage has been applied. As a result, for example, corona discharge takes place. This discharge produces ions. Ions having a positive charge are absorbed in the discharge electrode 1, while ions 4 having a negative charge are attracted by the counter electrode 2. As a result, an ionic wind occurs. In the course of attraction of negative ions by the counter electrode 2, the negative ions collide with or bind to floating matter 5 such as floating dust in the air, smoke from cigarette or cigar or tobacco, and harmful gas and carry them to the counter electrode 2.

As described later, the counter electrode 2 is an excellent gas absorbent material, and the carried floating matter 5 is efficiently adsorbed on this gas absorbent material, whereby air purification is carried out.

In the present invention, a plurality of discharge electrodes 1 and a plurality of counter electrodes 2 may be provided. The utilization of a plurality of discharge electrodes 1 and/or a plurality of counter electrodes 2 are sometimes advantageous from the viewpoint of purification efficiency.

### Gas absorbent material

In the air purifier according to the present invention, the gas absorbent material constituting the counter electrode comprises: a high-temperature carbonized charcoal which has been carbonized at a temperature of about 800°C or above; a low-temperature carbonized charcoal which has been carbonized at a temperature of about 500°C or below; and alginic acid or calcium oxide.

### (1) High-temperature carbonized charcoal and low-temperature carbonized charcoal

In the present invention, the high-temperature carbonized charcoal refers to a charcoal produced by carbonization at a temperature of about 800°C or above. In a preferred embodiment of the present invention, the high-temperature carbonized charcoal refers to a charcoal produced by carbonization at a temperature of about 800 to 1300°C, more preferably at a temperature of 900 to 1000°C. Activation, for example, activation by air or activation by steam, may be carried out in a refining process involved in the production of the high-temperature carbonized charcoal.

Further, in the present invention, the low-temperature carbonized charcoal refers to a charcoal produced by carbonization at a temperature of about 550°C or below. In a preferred embodiment of the present invention, the low-temperature carbonized charcoal refers to a charcoal produced by carbonization at a temperature of about 300 to 550°C, more preferably at a temperature of 450 to 500°C.

In the present invention, wood usable as a raw material for charcoal is not particularly limited and includes conifers such as cryptomerias, Japanese cypresses (hinoki), pines, and Japanese larches, bamboos and, in addition, building waste materials.

The high-temperature carbonized charcoal and the low-temperature carbonized charcoal are different from each other in absorptive capacity. Specifically, the high-temperature carbonized charcoal has high absorptive properties for formaldehyde, benzene, toluene, xylene, ethylbenzene, chlorobenzene and the like. On the other hand, the low-temperature carbonized charcoal has high absorptive properties for ammonia, amine and the like. A mixture of the high-temperature carbonized charcoal with the low-temperature carbonized charcoal has a good absorptive capacity for various materials.

The mixing ratio of the high-temperature carbonized charcoal to the low-temperature carbonized charcoal may be properly determined by taking into consideration the type of gas to be captured by the air purifier according to the present invention. However, the mixing ratio of the high-temperature carbonized charcoal to the low-temperature carbonized charcoal on a weight basis is preferably 30 : 70 to 60 : 40, more preferably 40 : 60 to 50 : 50.

Preferably, the high-temperature carbonized charcoal and the low-temperature carbonized charcoal are ground to a powder which is then used for contemplated applications. The particle diameter may be properly determined. The particle diameter, however, is preferably about 0.3 to 9.5 mm, more preferably about 0.6 to 1.18 mm.

Since the high-temperature carbonized charcoal has an electrical conductivity of not more than 10 Ω·cm³ in terms of volume resistivity, the absorbent material according to the present invention can also advantageously possess antistatic effect and electromagnetic radiation shielding effect. The low-temperature carbonized charcoal generally has a volume resistivity of about 10⁹ to 10¹² Ω·cm³.

### (2) Alginic acid and calcium oxide

In the present invention, alginic acid or its salt, for example, sodium, potassium, or calcium salt, binds charcoal particles to one another and, at the same time, has the effect of improving the gas absorptive capacity of the charcoal. When these alginic acids are used alone, as compared with the charcoal, the gas absorptive activity of the alginic acids is of course zero or, if any, is low. Therefore, it is surprising that the coexistence of the charcoal and the alginic acids can significantly improve the gas absorptive activity of the mixture of the high-temperature carbonized charcoal with the low-temperature carbonized charcoal.

In the present invention, the alginic acid or its salt refers to purified alginic acid or its salt and, in addition, materials composed mainly of alginic acid, for example, carageenan and chondrus.

Further, in the gas absorbent material used in the present invention, the amount of alginic acid or its salt added may be properly determined from the viewpoints of a function as a binder and an improvement in gas absorptive activity. The lower limit of the amount of alginic acid or its salt added, however, is preferably about 5% by weight, more preferably about 10% by weight. On the other hand, the upper limit of the amount of alginic acid or its salt added is preferably about 25% by weight, more preferably about 15% by weight.

In another embodiment of the present invention, the gas absorbent material used in the present invention comprises calcium oxide in addition to the high-temperature carbonized charcoal and the low-temperature carbonized charcoal. This calcium oxide, as with alginic acid, functions to improve the gas absorptive capacity of the high-temperature carbonized charcoal and the low-temperature carbonized charcoal. Further, by virtue of the presence of calcium oxide, the gas absorbent material can advantageously improve its fire resisting performance. Specifically, shell baked calcium, produced by baking shell, and quick lime may be utilized as calcium oxide.

Further, in the gas absorbent material used in the present invention, the amount of calcium oxide added may be properly determined from the viewpoint of improving the gas absorptive activity. The lower limit of the amount of calcium oxide added is preferably about 5% by weight, and the upper limit of the amount of calcium oxide added is preferably about 15% by weight, more preferably about 7% by weight.

Furthermore, in a further embodiment of the present invention, the gas absorbent material used in the present invention may include both alginic acid and calcium oxide. The addition of a combination of alginic acid with calcium oxide can further improve the gas absorptive activity of the high-temperature carbonized charcoal and the low-temperature carbonized charcoal.

In the present invention, the gas absorbent material used in the present invention may include a binder with the proviso that the above alginic acid is included. Binders usable herein include diatomaceous earth, cement, polymeric binders such as isocyanate resin emulsions, and starch pastes. For example, normal portland cement, moderate-heat portland cement, high early strength portland cement, blast furnace cement, and silica cement may be utilized as the cement. The utilization of diatomaceous earth or cement is advantageous in that the fire resisting performance of the gas absorbent material can be improved. Further, when diatomaceous earth or cement is utilized, the utilization of diatomaceous earth or cement in combination with a polymeric binder as a binder is preferred. The amount of the binder added may be properly determined. When the amount of the binder added is excessively large, however, the gas absorptive activity of the gas absorbent material is sometimes deteriorated. Therefore, preferably, the amount of the binder added is carefully determined. The amount of the binder added in the gas absorbent material according to the present invention is preferably about 10 to 40% by weight.

### (3) Production process

In the present invention, the gas absorbent material can easily be produced by pouring a mixture, prepared by mixing a high-temperature carbonized charcoal, a low-temperature carbonized charcoal, alginic acid or calcium oxide, optionally a binder, and other ingredients together, into a mold for molding, and drying the molding (at room temperature to 60°C or below for about 3 to 5 hr).

Further, in the production of the gas absorbent material, the utilization of a filler or a reinforcement is preferred from the viewpoint of enhancing the strength. Preferred examples of fillers or reinforcements usable herein include fibrous materials, such as Manila hemps and wood pulps, wire gauzes, and lattice or honeycomb structures.

The gas absorbent material used in the present invention may be in any form without particular limitation. Preferably, however, the gas absorbent material is in a board form. The board-shaped counter electrode may serve also as an indoor construction material, for example, a wall material or a ceiling material.

In a preferred embodiment of the present invention, a decorative paper may be applied to the surface of the gas absorbent material in a board form to improve the appearance. The decorative paper is not limited so far as it has such a certain level of air permeability that does not sacrifice the function of the gas absorbent material.

The gas absorbent material is basically electrically conductive and can be connected directly to the power supply.

### EXAMPLES

### Example 1: Preparation of high-temperature carbonized charcoal and low-temperature carbonized charcoal

A high-temperature carbonized charcoal and a low-temperature carbonized charcoal used in the following examples were prepared as follows. A concrete oven having an internal volume of 16 m³, which has a flame port in its front, has an exhaust port in its back lower part, and has been covered in its interior with refractory bricks, was provided. Wood was placed in the oven, and initial firing was carried out from the flame port. After the elapse of about 24 hr from the initial firing, decomposition and carbonization began, and a temperature of 300 to 400°C continued for additional about 48 hr. Thereafter, the temperature rose to 400 to 550°C. This temperature rise was judged to indicate the completion of carbonization and the initiation of a refining process. After the elapse of 5 to 10 hr from the initiation of the refining process, the oven was fully hermetically sealed and was cooled. The charcoal thus obtained was used as low-temperature carbonized charcoal. In the refining process, activation by air and activation by steam were carried out, the carbonization temperature was 900 to 1100°C, and this temperature was kept for 3 hr. The charcoal thus obtained was used as a high-temperature carbonized charcoal. The activation by air was carried out by forcibly feeding air into the oven at an air flow rate of 10 to 20 m³/min until the temperature reached about 900 to 1000°C. On the other hand, the activation by steam was carried out by forcibly feeding steam at a rate of 0.5 to 2 liters/min simultaneously with the force feeding of the air.

### Example 2: Production of gas absorbent material

A mixed charcoal powder (1000 g) composed of the high-temperature carbonized charcoal and the low-temperature carbonized charcoal prepared in Example 1 was mixed with 100 g of a Manila hemp to prepare a mixture. A pressure-sensitive adhesive (220 g) (composed of 60 g on a solid basis of vinyl acetate, 80 g of a starch paste, and 80 g of sodium alginate) and 200 g of shell baked calcium were added thereto.

Next, the mixture thus obtained was poured into a frame, followed by press molding to a given thickness. The whole assembly including the frame was then placed in a drying oven and was dried at room temperature or a temperature of 60°C or below for about 3 to 5 hr. The whole assembly was taken out of the drying oven, and the frame was then removed to prepare a board having a size of 300 × 400 × 15 mm which was then cut into a size of 200 × 150 × 15 mm for use as a gas absorbent material.

### Example 3: Air purifier and its performance test

The gas absorbent material prepared in Example 2 was used to construct an apparatus 10 shown in Fig. 2. Specifically, a box formed of a transparent plastic material (180 × 210 × 120 mm) for easy visual observation was provided. The gas absorbent material was placed on the bottom of the box and was used as a counter electrode 2. A discharge electrode 1 formed of a metallic needle was provided on the wall surface of this box. A d.c. high-voltage source 3 was connected to a position between the discharge electrode 1 and the counter electrode 2. The source 3 in its negative side was connected to the discharge electrode 1, and the source in its positive side was connected to the counter electrode 2 side.

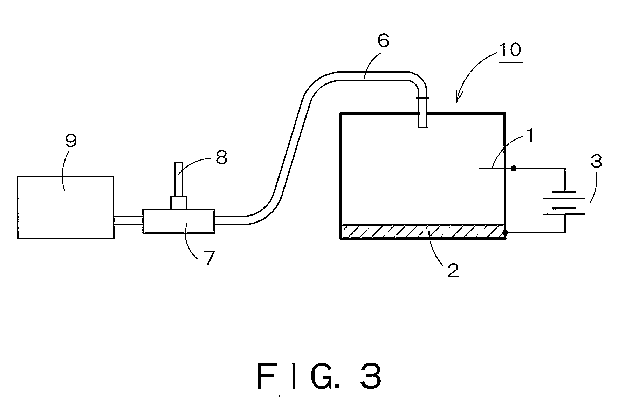

Further, as shown in Fig. 3, the apparatus 10 is constructed so that cigarette smoke is introduced through an introduction tube 6. A negative pressure generator 7 is connected to the introduction tube 6 and is constructed so that a cigarette 8 can be inserted into the negative pressure generator 7. Since air is guided from an compressed air source 9 to the negative pressure generator 7, smoke from the cigarette 8, together with the air, is introduced into the apparatus 10 through the introduction tube 6. The following experiment was carried out using this apparatus construction.

One cigarette (about 0.7 g) from which the filter was cut off was inserted into the negative pressure generator 7, and air was fed from the compressed air source 9 to introduce the smoke from the whole one cigarette into the apparatus 10 through the introduction tube 6. Immediately after that, a d.c. voltage of 8000 V was applied across the discharge electrode 1 and the counter electrode 2 to visually measure the time necessary for the smoke to disappear. In this experiment, the above procedure was repeated five times. Before and after the fifth experiment, the weight of the apparatus 10 from which the gas absorbent material had been removed was precisely measured, and the difference between the mass before the experiment and the mass after the experiment was determined as the amount of tar deposited on the plastic wall surface. The results were as shown in Table 1 below.

The procedure of the above experiment was repeated, except that the gas absorbent material was replaced by an aluminum plate having the same size as the gas absorbent material. The results were as shown in Table 1 below.

Separately, smoke from the whole one cigarette was introduced into the apparatus 10 without placing the gas absorbent material and the aluminum plate in the apparatus. As a result, the smoke did not disappear even after the elapse of 40 min from the introduction of the smoke.

**Table 1**

| Smoke disappearance time and amount of tar Material of counter electrode | | |
|---|---|---|
| | Gas absorbent material | Aluminum plate |
| 1st | 63 sec. | 97 sec. |
| 2nd | 66 sec. | 90 sec. |
| 3rd | 66 sec. | 111 sec. |
| 4th | 55 sec. | 100 sec. |
| 5th | 59 sec. | 99 sec. |
| Average | 61.8 sec. | 99.4 sec. |
| Amount of tar | 0.009 g | 0.030 g |

## Claims

1. An air purifier comprising a discharge electrode capable of producing ions upon discharge, a counter electrode located opposite to the discharge electrode, and a power supply capable of applying voltage between the discharge electrode and the counter electrode for inducing discharge for producing ions at the discharge electrode,
the counter electrode comprising a gas absorbent material comprising: a high-temperature carbonized charcoal which has been carbonized at a temperature of about 800°C or above; a low-temperature carbonized charcoal which has been carbonized at a temperature of about 500°C or below; and alginic acid or its salt or calcium oxide.

2. The air purifier according to claim 1, wherein the discharge on the discharge electrode is corona discharge.

3. The air purifier according to claim 1 or 2, wherein the gas absorbent material comprises both alginic acid or its salt and calcium oxide.

4. The air purifier according to any one of claims 1 to 3, wherein the mixing ratio of the high-temperature carbonized charcoal to the low-temperature carbonized charcoal in the gas absorbent material is 30 : 70 to 60 : 40 on a weight basis.

5. The air purifier according to any one of claims 1 to 4, wherein the high-temperature carbonized charcoal is produced by carbonization at a temperature of 800 to 1300°C.

6. The air purifier according to any one of claims 1 to 5, wherein the low-temperature carbonized charcoal is produced by carbonization at a temperature of 300 to 550°C.

7. The air purifier according to any one of claims 1 to 6, wherein the gas absorbent material functions also as an indoor construction material.
